(19) Europäisches Patentamt
European Patent Office
Office européen des brevets

(11) Veröffentlichungsnummer: **0 095 697**
**B1**

# (12) EUROPÄISCHE PATENTSCHRIFT

(45) Veröffentlichungstag der Patentschrift: **21.11.85**

(21) Anmeldenummer: **83105090.1**

(22) Anmeldetag: **24.05.83**

(51) Int. Cl.⁴: **C 07 C 61/39**, C 07 C 61/40, C 07 C 62/34, C 07 C 69/753, C 07 D333/24, C 07 D307/54, C 07 C 49/657, C 07 C 49/697, C 07 D333/22, C 07 D307/46, C 07 C 51/00

## (54) 2,2-Dimethyl-3-aryl-cyclo-butanone, ein Verfahren zu ihrer Herstellung sowie neue 2,2-Dimethyl-3-aryl-4-halogen-cyclobutanone.

(30) Priorität: **02.06.82 DE 3220730**

(43) Veröffentlichungstag der Anmeldung: **07.12.83 Patentblatt 83/49**

(45) Bekanntmachung des Hinweises auf die Patenterteilung: **21.11.85 Patentblatt 85/47**

(84) Benannte Vertragsstaaten: **BE CH DE FR GB LI**

(56) Entgegenhaltungen:
**EP-A- 0 043 492**
**CH-A- 589 584**
**DE-A- 2 654 062**
**DE-A- 2 715 336**

(73) Patentinhaber: **BAYER AG**
**Konzernverwaltung RP Patentabteilung**
**D-5090 Leverkusen 1 Bayerwerk (DE)**

(72) Erfinder: **Lantzsch, Reinhard, Dr.**
**Heymannstrasse 32**
**D-5090 Leverkusen 1 (DE)**
Erfinder: **Arlt, Dieter, Prof. Dr.**
**Rybnikerstrasse 2**
**D-5000 Köln 80 (DE)**
Erfinder: **Jautelat, Manfred, Dr.**
**Müllersbaum 28**
**D-5093 Burscheid (DE)**

EP 0 095 697 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2,2-Dimethyl-3-arylcyclobutanone, ein Verfahren zu ihrer Herstellung sowie neue 2,2-Dimethyl-3-aryl-4-halogen-cyclobutanone. Es handelt sich um Zwischenprodukte von weitgehend bekannten 2,2-Dimethyl-3-aryl-cyclopropan-carbonsäuren, welche ihrerseits als Zwischenprodukte zur Herstellung von insektizid wirksamen 2,2-Dimethyl-3-aryl-cyclopropan-carbonsäureestern verwendet werden können.

Es ist bekannt, daß man bestimmte 2,2-Dimethyl-3-aryl-cyclopropancarbonsäuren erhält, wenn man entsprechende 1-Aryl-2-methyl-propene mit Diazoessigsäureestern in Gegenwart von Katalysatoren umsetzt und die hierbei gebildeten 2,2-Dimethyl-3-aryl-cyclopropancarbonsäureester hydrolysiert (vgl. : Coll. Czech. Chem. Commun. *25* (1960), 1815). Diese Synthesemethode besitzt jedoch nur einen begrenzten Anwendungsbereich. Die Ausbeuten sind stark von der Art der Arylreste abhängig und in vielen Fällen unbefriedigend ; bei der Umsetzung von 1-Thienyl-2-methyl-propenen mit Diazoessigsäureestern werden nur Zersetzungsprodukte erhalten. Die Verwendung von Diazoessigsäureestern ist zudem mit den bekannten Sicherheitsrisiken behaftet. Ferner lassen sich die Ausgangsstoffe meist nur schwierig herstellen.

Weiter ist die Synthese von 2,2-Dimethyl-3-phenyl-cyclopropancarbonsäureethylester über 4-Methyl-3-phenyl-γ-valerolacton bekannt geworden (vgl. : Bull. Soc. Chim. France *1961,* 1857). Dieser Weg erfordert jedoch eine Reihe von zum Teil relativ komplizierten Vorstufen und liefert insgesamt nur eine geringe Ausbeute.

2,2-Dimethyl-3-phenyl-cyclopropancarbonsäureester erhält man auch durch katalytische Hydrierung von 2,2-Dimethyl-3-phenyl-cyclopropencarbonsäureestern (vgl. : Chem. Ber. *111* (1978). 3879). Die Synthese der als Ausgangsstoffe benötigten 2,2-Dimethyl-3-phenyl-cyclopropencarbonsäureester verläuft jedoch über mehrere, zum Teil sehr schwierige Stufen.

Ferner können 2,2-Dimethyl-3-aryl-cyclopropancarbonsäureester auch durch Umsetzung von Zimtsäureestern mit Triphenyl-isopropyl-phosphoniumiodid in Gegenwart starker Gasen, wie z. B. Butyllithium, hergestellt werden (vgl. : JP 8 105 435). Dieses Verfahren ist jedoch technisch aufwendig und teuer.

Die vorliegende Erfindung betrifft :

1) Ein Verfahren zur Herstellung von 2,2-Dimethyl-3-arylcyclobutanonen der Formel

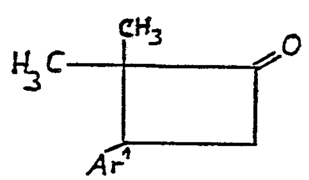

(IIIa)

in welcher

$Ar^1$ für Naphthyl oder für den Rest

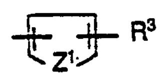

steht, worin

$Z^1$ für Sauerstoff, Schwefel, oder Ethen-1,2-diyl steht und

$R^3$ für Halogen, Cyano, Nitro, Trialkylsilyl oder für einen gegebenenfalls durch Halogen substituierten Rest aus der Reihe Alkyl, Cycloalkyl, Alkenyl, Alkoxy, Alkylendioxy, Alkylthio, Alkylsulfinyl, Alkylsulfonyl, Dialkylamino, Phenyl und Phenoxy steht.

Das neue Verfahren ist dadurch gekennzeichnet, daß man 1-Aryl-1-halogen-2,2-dimethyl-3-butanone der Formel (IV)

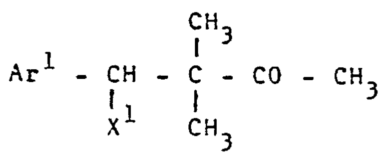

(IV)

in welcher

$Ar^1$ die oben angegebene Bedeutung besitzt und

$X^1$ für Chlor oder Brom steht, mit Basen in Gegenwart von Verdünnungsmitteln bei Temperaturen zwischen − 20 und + 150 °C umsetzt.

2) Neue 2,2-Dimethyl-3-aryl-cyclobutanone der Formel

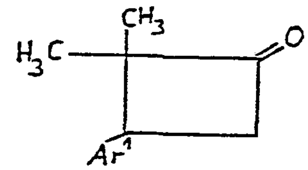

(IIIa)

in welcher $Ar^1$ die unter 1) angegebene Bedeutung besitzt.

3) Neue 2,2-Dimethyl-3-aryl-4-halogen-cyclobutanone der Formel (IIa)

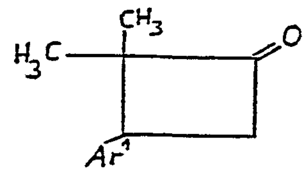

(IIa)

in welcher

$Ar^1$ die unter 1) angegebene Bedeutung besitzt und

X für Chlor oder Brom steht.

Die neuen 2,2-Dimethyl-3-aryl-4-halogen-cyclobutanone der Formel (IIa), in welcher $Ar^1$ und X die oben angegebene Bedeutung besitzen, werden hergestellt, indem man 2,2-Dimethyl-3-aryl-cyclobutanone der Formel (IIIa)

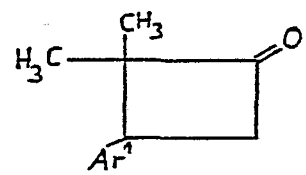

(IIIa)

in welcher $Ar^1$ die oben angegebene Bedeutung hat, mit Halogenen (Chlor oder Brom) in Gegenwart inerter Verdünnungsmittel bei Temperaturen zwischen − 30 und + 50 °C umsetzt ; Die neuen 2,2-Dimethyl-3-aryl-cyclobutanone sind durch Formel (IIIa) definiert. In dieser Formel steht vorzugsweise :

$Ar^1$ für Naphthyl oder für den Rest

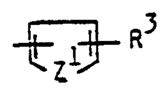

worin

$Z^1$ für Sauerstoff, Schwefel oder Ethen-1,2-diyl steht und

$R^3$ für Fluor, Chlor, Brom, Cyano, Nitro, Trimethylsilyl oder für gegebenenfalls durch Fluor und/oder Chlor substituierte Reste aus der Reihe $C_1$-$C_6$-Alkyl, $C_3$-$C_6$-Cycloalkyl, $C_2$-$C_6$-Alkenyl, $C_1$-$C_4$-Alkoxy, $C_1$-$C_2$-Alkylendioxy, $C_1$-$C_4$-Alkylthio, $C_1$-$C_4$-Alkylsulfinyl, $C_1$-$C_4$-Alkylsulfonyl, Di-$C_1$-$C_4$-alkylamino, Phenyl oder Phenoxy steht.

Besonders bevorzugt sind die neuen Verbindungen der Formel (IIIa), in welcher $Ar^1$ für in meta- und/oder in para-Position durch Reste aus der Reihe Fluor, Chlor, Brom, $C_1$-$C_4$-Alkyl, Trifluormethyl, Methoxy, Trifluormethoxy, Methylendioxy oder Difluormethylendioxy substituiertes Phenyl steht.

Als Beispiele für die neuen Verbindungen der Formel (IIIa) seien genannt :

2,2-Dimethyl-3-(4-fluor-phenyl)-, -3-(4-chlor-phenyl)-, -3-(4-methyl-phenyl)-3-(4-trifluormethyl-phenyl)-, -3-(4-tert.-butyl-phenyl)-, -3-(4-methoxy-phenyl)- und -3-(4-trifluormethoxyphl)-cyclobutanon.

Gemäß Chem. Comm. *1977*, 235 ist der Ringschluß zu den Cyclobutanonen nach den sogenannten Baldwin-Regeln nicht begünstigt (disfavoured) siehe dort Schema :

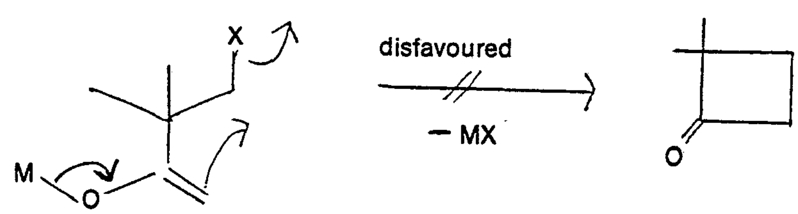

Übertragen auf das System der vorliegenden Anmeldung sollte die folgende Reaktionsfolge nicht begünstigt sein :

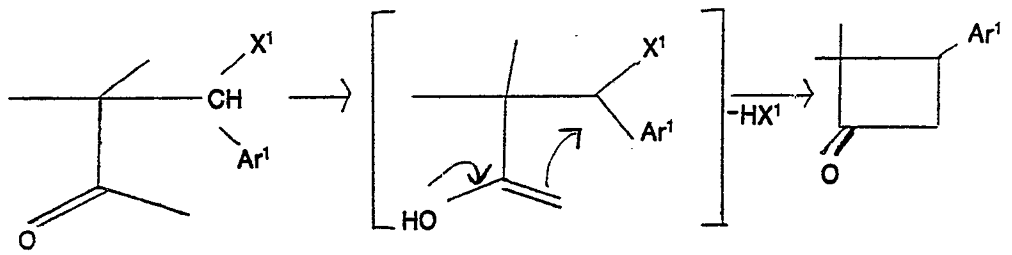


wobei $Ar^1$ und $X^1$ die im Anmeldungstext angegebene Bedeutung haben. Genau dies ist nun Gegenstand des Anspruchs 1).

Überraschenderweise kann man nun diese Umsetzung mit hoher Ausbeute durchführen.

Verwendet man bei dem oben unter 1) dargelegten Verfahren zur Herstellung der neuen 2,2-Dimethyl-3-aryl-cyclobutanone der Formel (IIIa) beispielsweise I-(4-Fluor-phenyl)-1-chlor-2,2-dimethyl-3-butanon und Kaliummethanolat als Ausgangskomponenten, so kann deren erfindungsgemäße Umsetzung durch das folgende Formelschema skizziert werden :

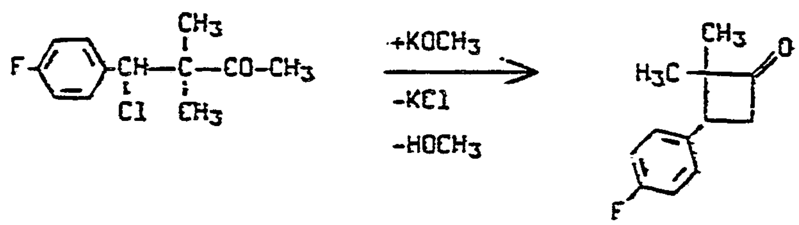


Das unter 1) dargelegte erfindungsgemäße Verfahren wird unter Verwendung von Verdünnungsmitteln durchgeführt. Als soche kommen die üblichen organischen Solventien und gegebenenfalls auch Wasser im Gemisch damit in Betracht. Vorzugsweise werden Alkohole, wie Methanol, Ethanol, n- und iso-Propanol sowie n-, iso-, sec- und tert.-Butanol als Verdünnungsmittel verwendet.

Als Basen können bei dem unter 1) dargelegten Verfahren Alkalimetall- und Erdalkalimetallhydroxide und -alkoholate verwendet werden. Vorzugsweise werden Alkalimetall-alkoholate, wie z. B. Natriummethanolat und -ethanolat oder Kaliummethanolat, -ethanolat und -tert.-butanolat eingesetzt.

Die Umsetzung wird bei Temperaturen zwischen – 20 und + 150 °C, vorzugsweise bei 0 bis + 100 °C und im allgemeinen bei Normaldruck durchgeführt.

Auf 1 Mol 1-Aryl-1-halogen-2,2-dimethyl-3-butanon der Formel (IV) setzt man im allgemeinen zwischen 1 und 2 Moläquivalente, vorzugsweise zwischen 1 und 1,2 Moläquivalente der zu verwendenden Base ein.

In einer bevorzugten Ausführungsform des unter 1) dargelegten Verfahrens wird die Base in Lösung vorgelegt und eine Lösung des 1-Aryl-1-halogen-2,2-dimethyl-3-butanons langsam dazugegeben. Nach Ende der Umsetzung wird nach üblichen Methoden aufgearbeitet, beispielsweise, indem man das Reaktionsgemisch mit Wasser verdünnt, neutralisiert, mit einem mit Wasser praktisch nicht mischbaren Lösungsmittel, wie z. B. Methylenchlorid, extrahiert, die Extraktionslösung trocknet und filtriert, das Filtrat einengt und den Rückstand im Hochvakuum destilliert.

Die als Zwischenprodukte benötigten 1-Aryl-1-halogen-2,2-dimethyl-3-butanone sind durch die Formel (IV) — oben unter 1) — allegemein definiert. In Formel (IV) haben die Reste $Ar^1$ und $X^1$ vorzugsweise bzw. besonders bevorzugt die gleichen Bedeutungen, wie sie oben bei der Definition der entsprechenden Reste $Ar^1$ und $X^1$ in formel (IIIa) als « vorzugsweise » bzw. « insbesondere bevorzugt » angegeben sind.

Als Beispiele für die Verbindungen der Formel (IV) seien genannt :

1-(4-Fluor-phenyl)-, 1-(4-Chlorphenyl)-, 1-(4-Methyl-phenyl)-, 1-(4-tert.-Butyl-phenyl)-, 1-(4-Trifluormethyl-phenyl)-, 1-(4-Methoxy-phenyl)- und 1-(4-Trifluormethoxy-phenyl)-1-chlor-2,2-dimethyl-3-butanon sowie 1-(4-Fluor-phenyl)-, 1-(4-chlor-phenyl)-, 1-(4-Methoxy-phenyl)-, 1-(4-tert.-Butyl-phenyl)-, 1-(4-Trifluormethylphenyl)-, 1-(4-Methoxy-phenyl)- und 1-(4-Trifluormethoxy-phenyl)-1-brom-2,2-dimethyl-3-butanon.

Man erhält die 1-Aryl-1-halogen-2,2-dimethyl-3-butanone der Formel (IV), wenn man 1-Aryl-2,2-dimethyl-3-butanone der Formel (V)

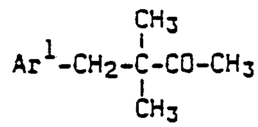

(V)

in welcher $Ar^1$ die oben angegebene Bedeutung hat,

in Gegenwart eines inerten Verdünnungsmittels, wie z. B. Tetrachlormethan, und in Gegenwart eines Katalysators, wie z. B. Azobisisobutyronitril, mit einem Mittel zur radikalischen Halogenierung, wie z. B. N-Brom-succinimid oder N-Chlor-succinimid bei Temperaturen zwischen 40 und 120 °C umsetzt. Die Produkte der Formel (IV) können durch Destillaton unter vermindertem Druck in reiner Form isoliert werden.

Die als Zwischenprodukte benötigten l-Aryl-2,2-dimethyl-3-butanone der Formel (V) erhält man, wenn man Aryl-methyl-halogenide der Formel (VI)

$$Ar^1—CH_2—X^2 \quad (VI)$$

in welcher

$Ar^1$ die oben angegebene Bedeutung hat und

$X^2$ für Halogen, vorzugsweise für Chlor steht,

mit Methyl-isopropylketon in Gegenwart einer Base, wie z. B. Kaliumhydroxid, in Gegenwart eines Verdünnungsmittels, wie z. B. Toluol, und in Gegenwart eines Phasentransferkatalysators, wie z. B. Tetrabutylammoniumbromid, bei Temperaturen zwischen 20 und 130 °C umsetzt und nach Filtrieren und Waschen der Reaktionsmischung die Produkte der Formel (V) durch Hochvakuum-Destillation isoliert.

Die Verbindungen der Formel IIa können mit Basen aus der Reihe der Alkalialkoholate in Gegenwart organischer Lösungsmittel oder Alkalimetall- und Erdalkalimetall- hydroxide und -carbonate in Gegenwart von Wasser und organischen Lösungsmitteln, gegebenenfalls in Gegenwart von Katalysatoren bei Temperaturen zwischen − 20 und + 100 °C zu 2,2-Dimethyl-3-aryl-cyclopropancarbonsäuren (carbonsäureestern) der Formel

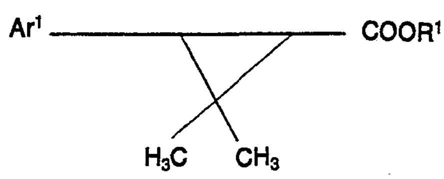

(I)

in welcher $Ar^1$ die oben genannte Bedeutung hat und $R^1$ für H oder $C_1$-$C_4$-Alkyl steht, umgesetzt werden.

Die vorgenannten 2,2-Dimethyl-3-aryl-cyclopropan-carbonsäuren der Formel (I) können als Zwischenprodukte zur Herstellung von insektizid wirksamen 2,2-Dimethyl-3-aryl-cyclopropancarbonsäureestern verwendet werden (vgl. : Coll. Czech. Chem. Commun. 25 (1960), 1815).

## Herstellungsbeispiele

### Beispiel 1

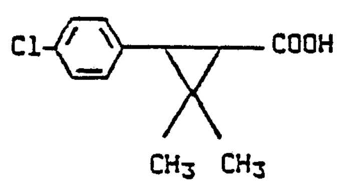

2,6 g 2,2-Dimethyl-3-(4-chlor-phenyl)-4-brom-cyclobutanon (Rohprodukt-Herstellung siehe Beispiel 2) werden in 10 ml Dioxan gelöst und diese Lösung wird zu einer Lösung von 2 g Natriumhydroxid in 20 ml Wasser und 10 ml Dioxan tropfenweise gegeben. Das Reaktionsgemisch wird 5 Minuten bei 20 °C gerührt und mit Methylenchlorid extrahiert. Nach Ansäuern der wässrigen Phase wird erneut mit Methylenchlorid extrahiert. Nach Abdestillieren des Lösungsmittels von den vereinigten Extraktionslösungen erhält man 1,1 g 2,2-Dimethyl-3-(4-chlor-phenyl)-cyclopropancarbonsäure vom Schmelzpunkt 115 °C.

Beispiel 2

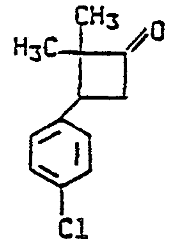

2,08 g (0,01 Mol) 2,2-Dimethyl-3-(4-chlor-phenyl)-cyclobutanon werden in 30 ml Chloroform gelöst und tropfenweise mit einer Lösung von 1,6 g Brom in 5 ml Chloroform versetzt. Nach einer Stunde Rühren bei 20 °C wird das Lösungsmittel im Wasserstrahlvakuum abdestilliert. Man erhält als Rückstand 2,6 g rohes 2,2-Dimethyl-3-(4-chlor-phenyl)-brom-cyclobutanon, welches ohne weitere Reinigung weiter eingesetzt wird (vgl. : Beispiel 1).

Beispiel 3

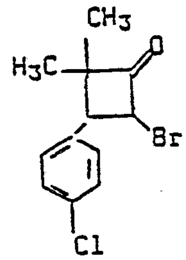

Man löst 0,91 g (0,04 Ml) Natrium in 100 ml Ethanol und gibt bei 20 °C 10,5 g (0,036 Mol) 1-Brom-1-(4-chlor-phenyl)-2,2-dimethyl-3-butanon in 20 ml Ethanol dazu. Das Reaktionsgemisch wird ca. 15 Stunden bei 20 °C gerührt, mit Wasser verdünnt und neutralisiert. Man extrahiert mit Methylenchlorid, trocknet mit Natriumsulfat und destilliert vom Filtrat im Wasserstrahlvakuum das Lösungsmittel ab. Der Rückstand wird durch Hochvakuum-Destillation gereinigt. Man erhält 4,8 g (65 % der Theorie) 2,2-Dimethyl-3-(4-chlorphenyl)-cyclobutanon vom Siedepunkt 98 bis 107 °C/0,05 mbar.

Beispiel 4

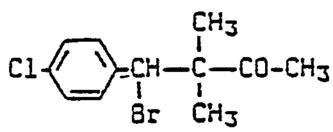

50 g (0,237 Mol) 1-(4-Chlor-phenyl)-2,2-dimethyl-3-butanon werden in 500 ml Tetrachlormethan gelöst und 44,5 g (0,25 Mol) N-Bromsuccinimid sowie 0,5 g Azobisisobutyronitril dazugegeben. Das Reaktionsgemisch wird unter Rückfluß zum Sieden erhitzt, bis beim Abstellen des Rührers keine feste Substanz mehr zu Boden sinkt. Nach dem Abkühlen wird vom Succinimid abgesaugt, vom Filtrat das Lösungsmittel im Wasserstrahlvakuum abdestilliert und der Rückstand im Hochvakuum destilliert. Man erhält 56,3 g (82 % der Theorie) 1-(4-Chlor-phenyl)-1-brom-2,2-dimethyl-3-butanon vom Siedepunkt 112 bis 118 °C/0,2 mbar.

Beispiel 5

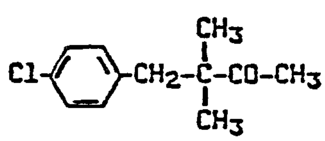

254 g (4 Mol) Kaliumhydroxid-Pulver (88 %-ig) werden in 1 l Toluol suspendiert. Hierzu werden 40 g Tetrabutylammoniumbromid und ein Gemisch aus 644 g (4 Mol) 4-Chlor-benzylchlorid und 430 g (5 Mol) Methyl-isopropylketon bei 85 °C langsam gegeben. Das Reaktionsgemisch wird noch 3 Stunden bei

85 °C gerührt. Nach dem Abkühlen wird filtriert und das Filtrat neutral gewaschen. Durch fraktionierte Hochvakuum-Destillation erhält man 732,5 g (87 % der Theorie) 1-(4-Chlor-phenyl)-2,2-dimethyl-3-butanon vom Siedepunkt 87 bis 90 °C/0,05 mbar.

**Patentansprüche**

1. Verfahren zur Herstellung von 2,2-Dimethyl-3-aryl-cyclobutanonen der Formel

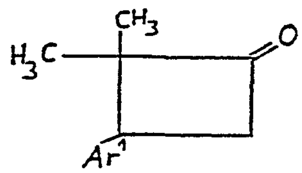

(IIIa)

in welcher $Ar^1$ für Naphthyl oder für den Rest

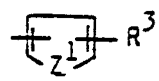

steht, worin

$Z^1$ für Sauerstoff, Schwefel, oder Ethen-1,2-diyl steht und

$R^3$ für Halogen, Cyano, Nitro, Trialkylsilyl oder für einen gegebenenfalls durch Halogen substituierten Rest aus der Reihe Alkyl, Cycloalkyl, Alkenyl, Alkoxy, Alkylendioxy, Alkylthio, Alkylsulfinyl, Alkylsulfonyl, Dialkylamino, Phenyl und Phenoxy steht, dadurch gekennzeichnet, daß man 1-Aryl-1-halogen-2,2-dimethyl-3-butanone der Formel (IV)

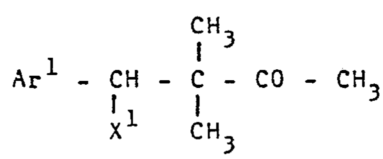

(IV)

in welcher

$Ar^1$ die oben angegebene Bedeutung besitzt und

$X^1$ für Chlor oder Brom steht, mit Basen in Gegenwart von Verdünnungsmitteln bei Temperaturen zwischen − 20 und + 150 °C umsetzt.

2. 2,2-Dimethyl-3-aryl-cyclobutanone der Formel

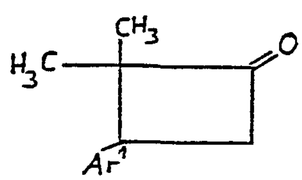

(IIIa)

in welcher $Ar^1$ die in Anspruch I) angegebene Bedeutung besitzt.

3. 2,2-Dimethyl-3-aryl-4-halogen-cyclobutanone der Formel (IIa)

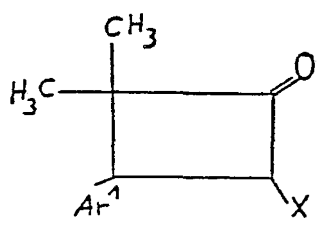

(IIa)

in welcher
Ar[1] die in Anspruch 1) angegebene Bedeutung besitzt und
X für Chlor oder Brom steht.

**Claims**

1. Process for the preparation of 2,2-dimethyl-3-aryl-cyclobutanones of the formula

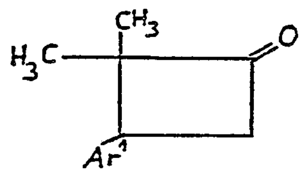

(IIIa)

in which Ar[1] represents naphthyl or the radical

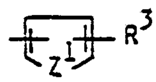

wherein
$Z^1$ represents oxygen, sulphur, or 1,2-ethenediyl and
$Z^3$ represents halogen, cyano, nitro, trialkylsilyl or an optionally halogen-substituted radical from the series comprising alkyl, cycloalkyl, alkenyl, alkoxy, alkylenedioxy, alkylthio, alkylsulphinyl, alkylsulphonyl, dialkylamino, phenyl and phenoxy, characterised in that 1-aryl-1-halogeno-2,2-dimethyl-3-butanones of the formula (IV)

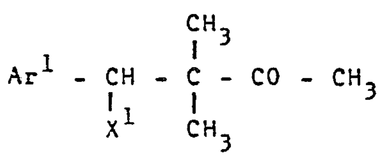

(IV)

in which
$Ar^1$ has the abovementioned meaning and
$X^1$ represents chlorine or bromine, are reacted with bases in the presence of diluents at temperatures between − 20 and + 150 °C.

2. 2,2-Dimethyl-3-aryl-cyclobutanones of the formula

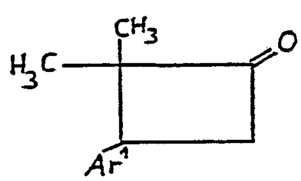

(IIIa)

in which $Ar^1$ has the meaning given in Claim 1.

3. 2,2-Dimethyl-3-aryl-4-halogeno-cyclobutanones of the formula (IIa)

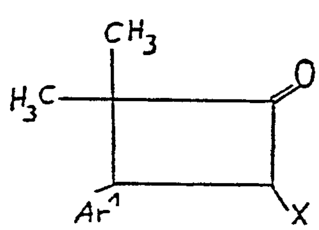

(IIa)

in which

Ar[1] has the meaning given in Claim 1 and

X represents chlorine or bromine.

**Revendications**

1. Procédé de production de 2,2-diméthyl-3-arylcyclobutanones de formule

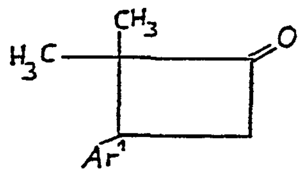

(IIIa)

dans laquelle Ar[1] est un groupe naphtyle ou le reste

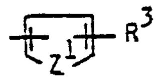

dans lequel

$Z^1$ représente l'oxygène, le soufre ou un groupe éthène-1,2-diyle et

$R^3$ représente un halogène, un groupe cyano, nitro, trialkylsilyle ou un reste éventuellement substitué par un halogène, de la série alkyle, cycloalkyle, alcényle, alkoxy, alkylènedioxy, alkylthio, alkylsulfinyle, alkylsulfonyle, dialkylamino, phényle et phénoxy, caractérisé en ce qu'on fait réagir des 1-aryl-1-halogéno-2,2-diméthyl-3-butanones de formule (IV)

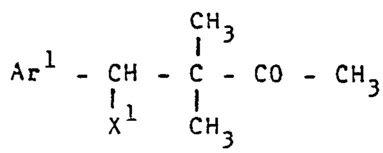

(IV)

dans laquelle

Ar[1] possède la définition indiquée ci-dessus et

$X^1$ représente le chlore ou le brome, avec des bases en présence de diluants à des températures comprises entre − 20 et + 150 °C.

2. 2,2-Diméthyl-3-arylcyclobutanones de formule

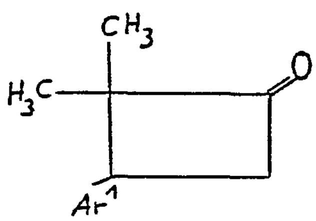

(IIIa)

dans laquelle Ar[1] possède la définition indiquée dans la revendication 1.

3. 2,2-Diméthyl-3-aryl-4-halogénocyclobutanones de formule (IIa)

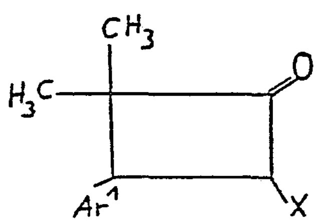

(IIa)

dans laquelle

Ar[1] possède la définition indiquée dans la revendication 1, et

X représente le chlore ou le brome.